(19) Europäisches Patentamt
European Patent Office
Office européen des brevets

(11) Publication number: **0 075 390**
**A1**

(12) # EUROPEAN PATENT APPLICATION

(21) Application number: **82304341.9**

(22) Date of filing: **17.08.82**

(51) Int. Cl.³: **C 07 C 45/45**, C 07 C 49/782, C 07 C 49/80, C 07 C 49/82

(30) Priority: **17.09.81 GB 8128178**

(43) Date of publication of application: **30.03.83**
**Bulletin 83/13**

(84) Designated Contracting States: **BE CH DE FR GB IT LI NL SE**

(71) Applicant: **IMPERIAL CHEMICAL INDUSTRIES PLC, Imperial Chemical House Millbank, London SW1P 3JF (GB)**

(72) Inventor: **Rose, John Brewster, "Uluvia" Pasture Road, Letchworth Hertfordshire (GB)**
Inventor: **Cinderey, Michael Bernard, 2 Swangley's Lane, Knebworth, Hertfordshire (GB)**

(74) Representative: **Sheller, Alan et al, Imperial Chemical Industries PLC Legal Department: Patents Thames House North Millbank, London SW1P 4QG (GB)**

(54) **Production of arylophenones.**

(57) Production of an arylophenone by reacting an aromatic carboxylic acid of formula $Ar(-CO_2H)_n$, where n is preferably 1 or 2, with an aromatic compound of formula Ar′–H to produce an arylophenone of formula $Ar(-CO-Ar')_n$, wherein the process is carried out in the presence of a fluoroalkane sulphonic acid, preferably trifluoromethane sulphonic acid. For example, 4,4′-dihydroxy-benzophenone is prepared from 4-hydroxybenzoic acid and phenol.

EP 0 075 390 A1

PRODUCTION OF ARYLOPHENONES

The present invention relates to a process for the production of an arylophenone.

Arylophenones are commercially useful substances and have been employed, for example, in the preparation of dyestuffs, polymers and ultraviolet light absorption agents.

Conventional prior art processes for making arylophenones include aromatic acylation using an aromatic acid chloride and a Friedel-Crafts catalyst such as $AlCl_3$, or an aromatic ester Fries rearrangement (also using a metallic halide catalyst such as $AlCl_3$) the ester having been formed from a phenol and an aromatic acid chloride.

It is also known to prepare arylophenones by aromatic acylation using an aromatic carboxylic acid, with liquid HF being employed as a condensing agent; such a process is described in British Patent 1 164 046.

The HF catalysed reactions are advantageous in that the carboxylic acids employed therein are usually cheaper and more readily available than the corresponding acid halides, and in that there are no metallic catalyst residues which might contaminate the resulting aromatic ketone. Nevertheless, the use of liquid HF is best avoided where possible because of its extremely corrosive properties and physiologically harmful action and the necessity of using pressure equipment.

It is also known from US Patent 3 073 866 to prepare hydroxy arylophenones by condensing an aromatic carboxylic acid with a phenol in the presence of $ZnCl_2$, $PCl_3$ and polyphosphoric acid. While the patent specifies that the arylophenones are produced in good yield and high purity using this process, it has been found that the product is in fact highly coloured and difficult to obtain in a pure state in high yield. The reaction also poses effluent

problems in that the contaminated phosphoric acid residue cannot be recovered economically but must be disposed of. Additionally, the presence of a metallic halide catalyst is undesirable for the reason given above.

We have now discovered a general process for making arylophenones involving aromatic acylation with an aromatic carboxylic acid which does not require the use of liquid HF or the $ZnCl_2/PCl_3$/polyphosphoric acid system as the condensing agent.

According to the present invention there is provided a process for the production of an arylophenone which comprises reacting an aromatic carboxylic acid of formula $Ar(-CO_2H)_n$ where Ar is an aromatic radical or a group containing aromatic radicals, $(-CO_2H)$ represents an aromatically-bound carboxylic acid group, and n is an integer, preferably 1 or 2, with an aromatic compound of formula Ar'-H where Ar' is an aromatic radical or a group containing aromatic radicals and -H is an aromatically-bound hydrogen atom, to produce an arylophenone of formula $Ar(-CO-Ar')_n$, wherein said process is carried out in the presence of a fluoroalkane sulphonic acid.

Thus the process of the invention employs a fluoroalkane sulphonic acid as the condensing agent instead of liquid HF or the $ZnCl_2/PCl_3$/polyphosphoric acid system. These sulphonic acids are liquids (or if solids are converted on melting into liquids) of high boiling point and do not require the use of pressure equipment. While being corrosive, they are far easier and safer to handle than liquid HF. Also their use does not incur the use of a metallic halide and so they are advantageous in this respect as well.

Preferred examples of fluoroalkane sulphonic acids which may be used are trifluoromethane sulphonic acid and difluoromethane sulphonic acid.

The process of the present invention appears to be a general one and applicable to the production of many arylophenones.

The group Ar of the aromatic carboxylic acid used in the process of the invention may be a single aromatic radical or a group containing two or more aromatic radicals; each two or more aromatic radicals may be connected by direct links and/or by non-aromatic radicals which do not deleteriously affect the condensation reaction. It is preferred that the maximum number of carboxylic acid substituents on any aromatic radical is two, and if Ar is a group containing two or more aromatic radicals, it is preferred that not more than two of the aromatic radicals are substituted by one or more carboxylic acid groups.

The aromatic radical(s) of the aromatic carboxylic acid may be nuclear unsubstituted (apart i.e. from possible aromatically-bound carboxylic acid groups) or have one or more nuclear substituents. The nuclear substituent(s) where present should be such that its position and nature does not deleteriously affect the process of the invention. Preferred nuclear substituents are hydroxyl, alkyl (containing 1 to 5 carbon atoms), alkoxy (containing 1 to 5 carbon atoms) and halogen (preferably Cl or F).

Preferred aromatic carboxylic acids that may be used in the present invention have the formulae

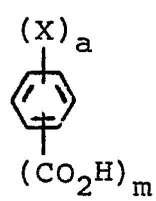

where a is 0, 1, 2, 3, 4 or 5 (preferably 0 or 1); and X is independently alkyl or substituted alkyl (preferably of 1 to 5 carbon atoms), alkoxy or substituted alkoxy (preferably of 1 to 5 carbon atoms), hydroxyl,

halogen (preferably F or Cl), aryl (preferably phenyl) including nuclear substituted aryl; m is 1 or 2; and $a+m \ngtr 6$; and

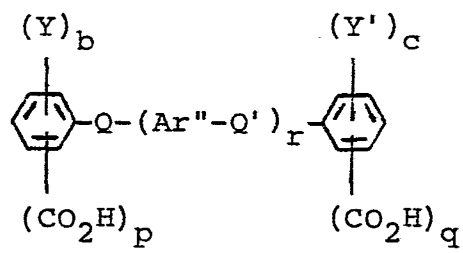

where Y and Y' are independently alkyl or substitued alkyl (preferably of 1 to 5 carbon atoms), alkoxy or substituted alkoxy (preferably of 1 to 5 carbon atoms), hydroxyl, halogen (preferably F or Cl), aryl (preferably phenyl) including substituted aryl; b and c are independently 0, 1, 2, 3 and 4 (preferably 0 or 1); p and q are independently 1 or 2 (preferably 1); $b+p \ngtr 5$ and $c+q \ngtr 5$; r is 0, 1, 2 or 3 (preferably 0 or 1); Q and Q' are independently a direct link, -CO-, $-SO_2-$, -O-, -S-, or $-CR_1R_2$ where $R_1$ and $R_2$ are independently hydrogen, alkyl (1 to 5 carbon atoms) or fluoroalkyl; Ar" is a divalent aromatic radical (preferably phenylene, biphenylene, or terphenylene).

Examples of aromatic carboxylic acids which may be used in the invention are: benzoic acid, 4-hydroxy-benzoic acid, 4-chloro-benzoic acid, 4-fluoro-benzoic acid, terephthalic acid, isophthalic acid, 4-carboxy-diphenyl, 4,4'-dicarboxy-diphenyl, 4,4'-dicarboxy-diphenyl ether, and 4-carboxyl-diphenyl ether.

The group Ar' of the aromatic compound Ar'-H may be a single aromatic radical or group containing two or more aromatic radicals; such two or more aromatic radicals may be connected by direct links or by non-aromatic radicals which do not deleteriously affect the condensation reaction.

The aromatic radical(s) of the compound Ar'-H may be nuclear unsubstituted or have one or more nuclear substituents. The nuclear substituent(s) where present should be such that its position and nature does not deleteriously affect the condensation reaction.

Preferred aromatic compounds Ar'-H have the formulae

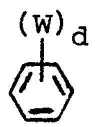

where d is 0, 1, 2, 3 or 4 (preferably 1 or 2), W is independently hydroxyl, halogen (preferably Cl or F), alkyl or substitued alkyl (preferably of 1 to 5 carbon atoms), alkoxy or substituted alkoxy (preferably of 1 to 5 carbon atoms), and aryl (preferably phenyl) including nuclear substitued phenyl; and

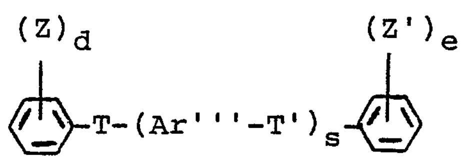

in which Z and Z' are independently alkyl or substituted alkyl (preferably of 1 to 5 carbon atoms), alkoxy or substituted alkoxy (preferably of 1 to 5 carbon atoms), hydroxyl, halogen (preferably F or Cl), aryl (preferably phenyl) including nuclear substituted aryl; d and e are independently 0, 1, 2, 3 (preferably 0 or 1); s is 0, 1, 2 or 3 (preferably 0 or 1); T and T' are independently a direct link, -CO-, $-SO_2-$, -O-, -S-, or $-CR_1R_2-$ where $R_1$ and $R_2$ are independently hydrogen, alkyl (1 to 5 carbon atoms) or fluoroalkyl; and Ar''' is a divalent aromatic radical (preferably phenylene, biphenylene or terphenylene).

Examples of aromatic compounds Ar'-H which may be used in the present invention are: benzene, phenol,

chlorobenzene, fluorobenzene, 4-hydroxy-diphenyl, 4-chloro-diphenyl, and 4-hydroxy-diphenyl ether. The process of the invention is particularly effective when the aromatic compound is a monohydric phenol, biphenylol, or naphthol.

If high yields of arylophenone are to be obtained in the process of the invention, it is preferable to employ substantially stoichiometric proportions of the aromatic carboxylic acid $Ar(-CO_2H)_n$ and aromatic compound Ar'-H. Thus if x moles of $Ar(-CO_2H)_n$ are used then preferably at least xn moles, and more preferably substantially xn moles, of Ar'-H should be employed.

It is preferable to employ at least one mole of fluoroalkane sulphonic acid per mole of Ar'-H. In many cases, however, it is useful to employ a large molar excess of the fluoroalkane sulphonic acid, e.g. 4 to 50 moles per mole of Ar'-H, so that it may act as a reaction solvent. Indeed, in some cases, the use of a very large excess of fluoroalkane sulphonic acid in order to achieve a very low reactant concentration (e.g. the use of $\geqslant$ 20 moles, particularly $\geqslant$ 40 moles of sulphonic acid per mole of aromatic compound), may be crucial to the success of the process.

Illustrative of arylophenones prepared by the process of the invention are:

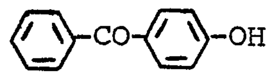

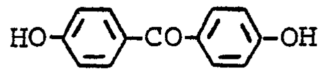

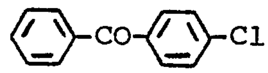

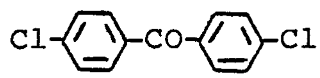

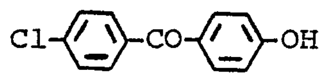

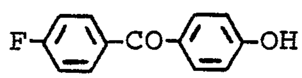

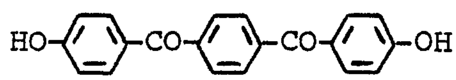

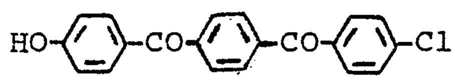

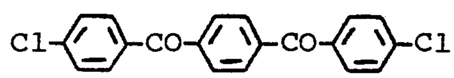

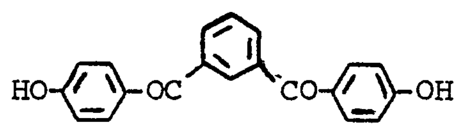

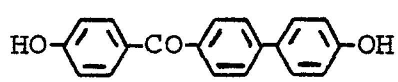

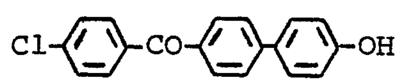

The mechanism of the process of the present invention is not entirely clear to us. It is apparent that when the nature of the reactants allows the formation of aromatic ester (as for example when reacting a phenol and a benzoic acid), the reaction (at least to a certain extent) proceeds through the ester as an intermediate and then undergoes a Fries-type rearrangement to form the arylophenone, and a notable feature of this ester rearrangement is that it proceeds primarily to yield the para rather than the ortho product. However, it seems that to a certain extent the reaction also proceeds in

such cases via direct (i.e. one-stage) nuclear acylation. It is believed that the greater the reactant concentration in the process, the greater is the tendency to proceed through a para Fries-type rearrangement via the aromatic ester intermediate, although there should preferably still be at least one mole of fluoroalkane sulphonic acid per mole of Ar'-H (i.e. a non-catalytic amount). It is also evident that where the reactants cannot form an aromatic ester intermediate (as for example when reacting a chlorobenzene with a benzoic acid), the reaction cannot proceed through a Fries-type rearrangement and it is presumed that direct nuclear acylation must be occurring.

The present invention is now illustrated by the following examples.

EXAMPLE 1

Benzoic acid (0.0244 g; 0.2 millimole) and phenol (0.0188 g; 0.2 millimole) were weighed into a small sample tube and trifluoromethane sulphonic acid (0.7 ml; about 8 millimole) added thereto. The tube was stoppered, shaken at ambient temperature until the reagents had dissolved and the resulting solution transferred quickly to a tube used for nuclear magnetic resonance (nmr) spectroscopy. Nmr spectra were run after increasing periods of time; when the tube was not being used in the 60 MHz nmr spectrometer, it was stored in a large test tube at ambient temperature under a slow stream of dry nitrogen. The results were as follows:

| | |
|---|---|
| 1/1 (after 1 hour) | - nmr spectrum consistent with 4-hydroxy-benzophenone (using nmr spectrum of product as comparison), although some impurities probably present; solution yellow. |
| 1/2 (after 6 hours) | - very clean nmr spectrum, identical with that of 4-hydroxy-benzophenone; solution orange. |
| 1/3 (after 7 days) | - no change in nmr spectrum; solution rosé. |

On this basis, it was concluded that a clean reation to 4-hydroxy-benzophenone had taken place in a reaction time of less than 1 hour at ambient temperature.

EXAMPLE 2

The procedure of Example 1 was repeated, but employing 4-hydroxybenzoic acid (0.0276 g; 0.2 millimole), phenol (0.0188 g; 0.2 millimole) and trifluoromethane sulphonic acid (0.7 ml; about 8 millimole). The results were as follows:

2/1 (after 0.5 hours) - nmr spectrum virtually identical with a standard spectrum of 4,4'-dihydroxy-benzophenone with little impurity present; solution pale yellow.

2/2 (after 3 hours) - no change and completely clean nmr spectrum; solution yellow.

2/3 (after 8 days) - no change; solution yellow

On this basis, it was concluded that a clean reaction to 4,4'-dihydroxy-benzophenone had taken place in a reaction time of less than 0.5 hours at ambient temperature.

EXAMPLE 3

The procedure of Example 1 was repeated but employing 4-chloro-benzoic acid (0.0313 g; 0.2 millimole), phenol (0.0188 g; 0.2 millimole) and trifluoromethane sulphonic acid (0.7 ml; about 8 millimole). The results were as follows:

3/1 (after 0.5 hours) - a marked change has taken place but clearly a significant amount of 4-chloro-benzoic acid still present; solution yellow.

3/2 (after 29.5 hours)- nmr spectrum virtually identical with a standard spectrum of 4-chloro-4'-hydroxy-benzophenone; solution greenish yellow.

3/3 (after 8 days) - no change.

On this basis, it was concluded that a clean reaction to 4-chloro-4'-hydroxy-benzophenone had taken place in a reaction time of less than 1 day at ambient temperature.

EXAMPLE 4

The procedure of Example 1 was repeated but employing terephthalic acid (0.0332 g; 0.2 millimole), phenol (0.0376 g; 0.4 millimole) and trifluoromethane sulphonic acid (0.7 ml; about 8 millimole). The results were as follows:

4/1 (after 0.5 hours) - little if any change has taken place (nmr spectrum that of mixture of starting reagents).

4/2 (after 19 hours) - a marked change has taken but starting material is still perhaps evident; solution rosé.

4/3 (after 6 days) - nmr spectrum virtually identical with a standard spectrum of 1,4-bis(4-hydroxybenzoyl) benzene; solution rosé.

4/4 (after 12 days) - no change.

On this basis it was concluded that a clean reaction to 1,4-bis(4-hydroxybenzoyl) benzene had taken place in a reaction time of less than 6 days at ambient temperature.

EXAMPLE 5

The procedure of Example 1 was repeated, but employing 4,4'-dicarboxy-diphenyl ether (0.0260 g; 0.1 millimole), phenol (0.0188 g; 0.2 millimole) and trifluoromethane sulphonic acid (0.7 ml). The results were as follows:

5/1 (after 0.25 hours) - a marked changed has taken place; yellow solution.

5/2 (after 5 hours) - a further change has taken place; yellow solution.

5/3 (after 6 days) - nmr spectrum now consistent with that expected of 4,4'-bis(4-hydroxybenzoyl)-diphenyl ether; solution yellow.

On this basis, it was concluded that a clean reaction to 4,4'-bis(4-hydroxybenzoyl)-diphenyl ether had taken place in a reaction time of less than 6 days at ambient temperature.

EXAMPLE 6

The procedure of Example 1 was repeated, but employing benzoic acid (0.0244 g; 0.2 millimole), chlorobenzene (0.0225 g; 0.2 millimole) and trifluoromethane sulphonic acid (0.7 ml; about 8 millimole). The results were as follows:

| | |
|---|---|
| 6/1 (after 0.25 hours) | - no change has taken place (only starting materials present). |
| 6/2 (after 5.5 hours) | - no change. |
| 6/3 (after 7 days) | - all original benzoic acid is present but chlorobenzene has disappeared (leaked through tube cap?); more chlorobenzene added. |
| 6/4 (after 2 hours) | - no product although 40% deficiency in chlorobenzene. |
| 6/5 (after 29.5 hours) | - no change, so tube heated to 50-55°C under nitrogen; yellow solution. |
| 6/6 (after 30.5 hours at 50°C) | - a slight reaction indicated although more chlorobenzene lost; more chlorobenzene added and tube heated to 100°C. |
| 6/7 (after 22.5 hours at 100°C) | - a marked change has taken place; solution dark brown. |
| 6/8 (after 5 days at 100°C) | - little further change; nmr spectrum is very similar to a standard spectrum of 4-chlorobenzophenone although impurities are present (estimated at about 10%); solution very dark brown. |

On this basis, it was concluded that a somewhat incomplete reaction to 4-chloro-benzophenone had occurred after about 1 day at 100°C.

EXAMPLE 7

The procedure of Example 1 was repeated, but employing 4-hydroxy-benzoic acid (0.0276 g; 0.2 millimole), chlorobenzene (0.0225 g; 0.2 millimole) and trifluoromethane sulphonic acid (0.7 ml; about 8 millimole). The results were as follows (little or no reaction occurred at ambient temperature or 50°C as in Example 6, so the tube was heated to 100°C):

| | |
|---|---|
| 7/1 (after 23.5 hours at 100°C) | - a marked change has taken place; orange brown solution. |
| 7/2 (after 5 days at 100°C) | - a further change has occurred to give a much simpler nmr spectrum similar to that of a standard spectrum of 4-chloro-4'-hydroxy benzophenone (indicating perhaps a 50% yield). |

On this basis it was concluded that a somewhat incomplete reaction to 4-chloro-4'-hydroxy benzophenone had occurred after 5 days at 100°C, with probably not more than about a 50% yield of this ketone being produced.

EXAMPLE 8

The procedure of Example 1 was repeated but employing 4-chloro-benzoic acid (0.0313 g; 0.2 millimole), chlorobenzene (0.0225 g; 0.2 millimole), and trifluoromethane sulphonic acid (0.7 ml; about 8 millimole). The results were as follows (little or no reaction occurred at ambient temperature or 50°C as in Example 6, so the tube was heated to 100°C):

| | |
|---|---|
| 8/1 (after 23 hours at 100°C) | - nmr spectrum completely changed and similar to a standard spectrum of 4,4'-dichloro-benzophenone but with some impurities (about 25%) present; soluton dark green-brown. |

8/2 (after 5 days at 100°C) - little change; dark orange-brown solution.

It was concluded that a somewhat incomplete reaction to 4,4'-dichloro-benzophenone had occurred after one day at 100°C.

EXAMPLE 9

The procedure of Example 1 was repeated, but employing terephthalic acid (0.0332 g; 0.2 millimole), chlorobenzene (0.045 g; 0.4 millimole) and trifluoromethane sulphonic acid (0.7 ml; about 8 millimole). The results were as follows (little or no reaction occurred at ambient temperature as in Example 6, although some reaction was occurring at 50°C; the tube was heated to 100°C as in Example 6):

9/1 (after 22 hours at 100°C) - completely changed spectrum which indicates 1,4-bis(4-chlorobenzoyl)-benzene with about 25% of impurities (by comparison with a standard spectrum); yellow-brown solution.

9/5 (after 5 days at 100°C) - little change; very dark orange-brown solution.

It was concluded that a somewhat incomplete reaction to 1,4-bis(4-chlorobenzoyl)-benzene had occurred after one day at 100°C.

EXAMPLE 10

The procedure of Example 1 was repeated, but employing 4-hydroxy-benzoic acid (0.0414 g; 0.3 millimole), 4-hydroxy-diphenyl (0.051 g; 0.3 millimole) and trifluoromethane sulphonic acid (0.7 ml; about 8 millimole). The results were as follows (reaction at ambient temperature):

11/1 (after 0.25 hours) - nmr spectrum indicates that the reaction is well underway; orange solution.

11/2 (after 23.5 hours) - completely changed to a product having an nmr spectrum consistent with 4-hydroxy-4'-(4-hydroxybenzoyl)-diphenyl (standard not available for comparison).

It was concluded that a clean reaction to 4-hydroxy-4'-(4-hydroxybenzoyl)-diphenyl had occurred in less than one day at ambient temperature.

EXAMPLE 11

The procedure of Example 1 was repeated, but employing 4-chloro-benzoic acid (0.047 g; 0.3 millimole), 4-hydroxy-diphenyl (0.051 g; 0.3 millimole) and trifluoromethane sulphonic acid (0.7 ml; about 8 millimole). The results were as follows (reaction at ambient temperature):

12/1 (after 0.25 hours) - nmr spectrum indicates that the reaction is well underway; orange-brown solution.

12/2 (after 8 hours) - completely changed to a product having an nmr spectrum essentially identical with that of a standard spectrum of 4-hydroxy-4'-(4-chlorobenzoyl)-diphenyl; very dark orange-brown solution.

12/3 (after 4 days) - little change.

It was concluded that a clean reaction to 4-hydroxy-4'-(4-chlorobenzoyl)-diphenyl had occurred in less than 8 hours at ambient temperature.

EXAMPLE 12 (Comparative)

Example 2 was repeated (on a 0.4 millimole scale) but using 98% w/w $H_2SO_4$ instead of trifluoromethane

sulphonic acid. Spectra were taken after 0.25 hours, 6.5 hours, 4 days, 12 days and 15 days. No 4,4'-dihydroxy-benzophenone could be detected at any stage; instead both starting reagents became sulphonated. Repeat runs (at 50-60°C) produced the same result.

EXAMPLE 13

The following were charged to a 150 ml 3-necked flask provided with an addition funnel, nitrogen flow, air condenser and stirrer. isophthalic acid (6.22 g; 0.0375 mole), phenol (7.05 g; 0.075 mole) and trifluoromethane sulphonic acid (60 ml; about 0.7 mole). The pale yellow solution was heated with stirring to 70°C, when it became deep red. 12 ml samples were taken at this temperature (stirring being continued) after 2 hours, 5 hours, 25 hours and 100 hours. Each sample was worked up by adding dropwise to 150 ml of stirred demineralised water in a beaker. The mixture was heated to boiling and after a few minutes the product was filtered off, washed once with warm water, then thrice with hot water, and finally dried at 120°C overnight. The product samples so formed were yellow in colour. Each sample was examined by infra-red (ir) and nmr spectroscopy and its melting point taken. The results were as follows:

| | |
|---|---|
| 14/1 (after 2 hours at 70°C) | - melting point about 184°C; ir and nmr spectra indicate product is mostly 1,3-bis(4-hydroxybenzoyl) benzene (by comparison with spectra of authentic product) although a little impurity is present. |
| 14/2 (after 5 hours at 70°C) | - melting point about 190°C; ir and nmr spectra indicate product is 1,3-bis(4-hydroxybenzoyl)-benzene with only very little impurity. |

<u>14/3 (after 25 hours at 70°C)</u> - melting point about 194°C, ir and nmr spectra unchanged (no appreciable impurity).

<u>14/4 (after 100 hours at 70°C)</u> - melting point about 194°C; ir and nmr spectra unchanged.

It was concluded that quite a clean reaction to 1,3-bis(4-hydroxybenzoyl)-benzene had occurred in about 2 hours at 70°C.

<u>EXAMPLE 14</u>

The procedure of Example 13 was repeated, but employing terephthalic acid (8.3 g; 0.05 mole), phenol (9.4 g; 0.10 mole) and trifluoromethane sulphonic acid (60 ml; about 0.7 mole). 12 ml samples were taken after 1 hour, 5 hours, and 25 hours at 70°C, and then a further 12 ml sample was taken after 4 hours at 120°C. The samples were worked up and evaluated in a similar manner to those of Example 14; the product samples were yellow powders. The results were as follows:

<u>15/1 (after 1 hour at 70°C)</u> - melting point 260-275°C; ir and nmr spectra indicate product is mostly 1,4-bis(4-hydroxybenzoyl)-benzene (by comparison with spectra of authentic product) although some impurity is present.

<u>15/2 (after 5 hours at 70°C)</u> - melting point 265-290°C, ir and nmr spectra indicate product is essentially 1,4-bis(4-hydroxybenzoyl)-benzene with a little impurity.

<u>15/3 (after 25 hours at 70°C )</u> - melting point 265-290°C; ir and nmr spectra as for 15/2.

<u>15/4 (after 4 hours at 120°C)</u> - melting point 275-295°C; ir and nmr spectra as for 15/2.

It was concluded that a fairly clean reaction to 1,4-bis(4-hydroxybenzoyl) benzene had occurred in less than 5 hours at 70°C.

EXAMPLE 15

The following were changed to a 100 ml three-necked flask provided with an addition funnel, nitrogen flow, air condenser and stirrer: 4-fluoro-benzoic acid (14.01 g; 0.1 mole), and phenol (9.41 g; 0.1 mole). The solid mixture was cooled to -10°C and trifluoromethane sulphonic acid (50 ml) added with stirring. The temperature of the reaction mixture rose to 32°C and cooling was applied to reduce it to 15°C. The flask and its contents were left overnight at ambient temperature. The reaction mass was poured into 800 ml demineralised water to yield a solid product which was filtered off and washed twice with water. The product was slurried with 200 ml water and stirred for 0.5 hours. The product was again filtered and washed twice with water. The slurrying/washing procedure was repeated and the resulting solid sucked dry on the filter and finally dried at 80°C overnight. The product had a melting point of 156°C and its nmr and ir spectra showed it to be 4'-hydroxy-4-fluoro-benzophenone. The yield of product was 16.7 g (77%). Recrystallisation from ethanol/water gave 12.7 g of a pale pinky brown powder of melting point 166.5-168.5°C.

EXAMPLE 16

The procedure of Example 15 was essentially repeated except that the reactants were 4-hydroxy-benzoic acid (13.81 g; 0.1 mole) and phenol (9.44 g; 0.1 mole). The product obtained was a yellow/cream-coloured powder having melting point 217°C; its ir and nmr spectrum showed it to be 4,4'-dihydroxy-benzophenone; the yield was 19.9 g (93%). Recrystallisation gave 19 g of an off-white powder of melting point 218°C.

EXAMPLE 17

Using the apparatus of Example 15, the following were charged to the flask: terephthalic acid (11.66 g; 0.07 mole); and phenol (13.18 g; 0.14 mole). The solid mixture was cooled to -10°C and trifluoromethane sulphonic acid (50 ml) added with stirring. The temperature of the reaction mixture was allowed to rise to ambient temperature and then, after 0.5 hour, was raised to 50°C and maintained there for 23 hours. A further 25 ml of trifluoromethane sulphonic acid were added and the temperature increased to 70°C. After being kept for 4 hours at 70°C, the reaction mixture was cooled to room temperature and then added dropwise into 400 ml demineralised water. The mixture was stirred for 22 hours and the product filtered off. The solid was slurried with 400 ml water, left over the week-end, and then filtered. The filtered product was recrystallised from 400 ml methylated spirits/100 ml water. The resulting crystals were filtered off, washed and then dried overnight at 150°C. The product so obtained had a melting point of 292°C; its yield was 14.9 g. The ir spectrum of the product was consistent with 1,4-bis(4-hydroxybenzoyl)-benzene although the nmr spectrum indicated impurities to be present that appeared to be consistent with the mono- or di-esters of terephthalic acid (this is quite unlike the nmr-scale reaction of Example 4 where the reactant concentration was much lower).

EXAMPLE 18

The procedure of Example 17 (up until work-up) was essentially repeated except that the reactants were isophthalic acid (11.63 g; 0.07 mole) and phenol (13.18 g; 0.14 mole). The reaction mixture was poured into 400 ml of demineralised water to yield a precipitate which was filtered off and then repeatedly reslurried in water, filtered and washed and finally dried overnight at 100°C. The yield of product was 10.76 g (about 50%).

Recrystallisation gave a melting point of 193-200°C. The ir and nmr spectra showed the product to be 1,3-bis(4-hydroxybenzoyl)-benzene although the nmr spectrum indicated that a certain proportion of impurity was present (unlike the nmr-scale reaction of Example 13), probably the mono- or diester of isophthalic acid.

EXAMPLE 19

Using the apparatus of Example 15, the following were charged to the flask: 4-hydroxy-benzoic acid (9.517 g; 0.0689 mole); and 4-hydroxy-diphenyl (11.72 g; 0.0689 mole). The solid mixture was cooled to -15°C and trifluoromethane sulphonic acid (50 ml) added with stirring. A dark red solution was obtained after 0.5 hour. The temperature was allowed to rise to ambient overnight and then the solution was heated to 50°C; after about 3.5 hours at 50°C, a further 25 ml of trifluoromethane sulphonic acid was added and the temperature raised to 65°C for about 1.5 hours. The reaction mixture was added dropwise to 800 ml demineralised water to precipitate the product. After washing and drying, the yield of product was 18.8 g (95%). Recrystallisation from methylated spirits gave 13.5 g of a product of melting point 190-198°C. The ir spectrum indicated the product to be 4-hydroxy-4'-(4-hydroxybenzoyl)-diphenyl although the nmr spectrum indicated the presence of a considerable amount of ester as an impurity.

EXAMPLE 20

The procedure of Example 18 was repeated except that the reactants were 4-chloro-benzoic acid (10.15 g; 0.0648 mole) and 4-hydroxy-diphenyl (11.02 g; 0.0648 mole). The yield of crude product obtained was 19.5 g (97%); its melting point (after recrystallisation) was 196-197°C. The ir and nmr spectra of the product showed it to be 4-hydroxy-4'-(4-chlorobenzoyl)-diphenyl of high purity.

CLAIMS

1. A process for the production of an arylophenone which comprises reacting an aromatic carboxylic acid of formula $Ar(-CO_2H)_n$ where Ar is an aromatic radical or a group containing aromatic radicals, $(-CO_2H)$ represents an aromatically-bound carboxylic acid group, and n is an integer, with an aromatic compound of formula Ar'-H where Ar' is an aromatic radical or a group containing aromatic radicals and -H is an aromatically-bound hydrogen atom, to produce an arylophenone of formula $Ar(-CO-Ar')_n$, wherein said process is carried out in the presence of a fluoroalkane sulphonic acid.

2. A process according to claim 1 wherein the fluoroalkane sulphonic acid is trifluoromethane sulphonic acid or difluoromethane sulphonic acid.

3. A process according to claim 1 or claim 2 wherein the aromatic carboxylic acid used has the formula

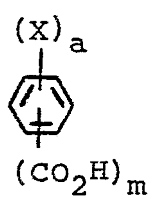

where a is 0, 1, 2, 3, 4 or 5; and X is independently alkyl or substituted alkyl, alkoxy or substituted alkoxy, hydroxyl, halogen, aryl, including nuclear substituted aryl; m is 1 or 2; and $a+m \ngtr 6$.

4. A process according to claim 3 wherein in the aromatic carboxylic acid used a is 0 or 1.

5. A process according to either claim 1 or claim 2 wherein the aromatic carboxylic acid used has the formula

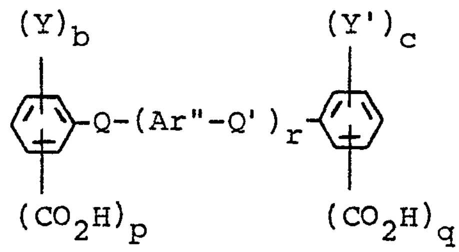

where Y and Y' are independently alkyl or substituted alkyl, alkoxy or substituted alkoxy, hydroxyl, halogen, aryl including nuclear substituted aryl; b and c are independently 0, 1, 2, 3 or 4; p and q are independently 1 or 2; $b+p \ngtr 5$ and $c+q \ngtr 5$; r is 0, 1, 2 or 3; Q and Q' are independently a direct link, -CO-, $-SO_2-$, -O-, -S- or $-CR_1R_2-$ where $R_1$ and $R_2$ are independently hydrogen, alkyl (1 to 5 carbon atoms) or fluoroalkyl; and Ar" is a divalent aromatic radical.

6. A process according to claim 5 wherein in the aromatic carboxylic acid used b and c are independently 0 or 1, p and q are 1, and r is 0 or 1.

7. A process according to any one of the preceding claims wherein the aromatic compound Ar'-H used has the formula

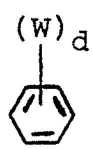

where d is 0, 1, 2, 3 or 4, W is independently hydroxyl, halogen, alkyl or substituted alkyl, alkoxy or substituted alkoxy and aryl including nuclear substituted aryl.

8. A process according to claim 7 wherein in the aromatic compound used d is 1 or 2.

9. A process according to any one of claims 1 to 6 wherein the aromatic compound Ar'-H has the formula

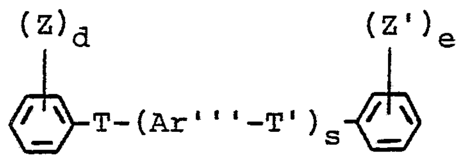

where Z and Z' are independently alkyl or substituted alkyl, alkoxy or substituted alkoxy, hydroxy, halogen, aryl including nuclear substituted aryl; d and e are independently 0, 1, 2 or 3; s is 0, 1, 2 or 3 ; T and T' are independently a direct link, -CO-, $-SO_2-$, -O-, or $-CR_1R_2-$ where $R_1$ and $R_2$ are independently hydrogen, alkyl (1 to 5 carbon atoms) or fluoroalkyl; and Ar''' is a divalent aromatic radical.

10. A process according to claim 9 wherein in the aromatic compound used d and e are independently 0 or 1 and s is 0 or 1.

11. A process according to any one of the preceding claims wherein substantially stoichiometric proportions of the aromatic ketone $Ar(-CO_2H)_n$ and aromatic compound Ar'-H are employed, i.e. substantially n moles of aromatic compound Ar'-H per mole of aromatic ketone $Ar(-CO_2H)_n$.

12. A process according to any one of the preceding claims wherein at least one mole of fluoroalkane sulphonic acid per mole of aromatic compound is employed.

13. A process according to claim 12 wherein sufficient fluoroalkane sulphonic acid to act as a reaction solvent ——————— is employed.

14. An arylophenone when produced by a process according to any one of the preceding claims.

European Patent Office

# EUROPEAN SEARCH REPORT

0075390

Application number

EP 82304341.9

| Category | Citation of document with indication, where appropriate, of relevant passages | Relevant to claim | CLASSIFICATION OF THE APPLICATION (Int. Cl. 3) |
|---|---|---|---|
| X | GB - A - 1 378 913 (BAYER)<br>* Claims 1-15 *<br>-- | 1-14 | C 07 C 45/45<br>C 07 C 49/782<br>C 07 C 49/80<br>C 07 C 49/82 |
| P,X | EP - A1 - 0 057 503 (ICI) (11-08-1982)<br>* Claim 1 *<br>-- | 1 | |
| A | US - A - 3 907 837 (F. EFFENBERGER et al.)<br>* Claims 1,2 *<br>-- | 1 | |
| D,A | GB - A - 1 164 046 (KOPPERS)<br>* Claim 1 *<br>-- | 1 | |
| D,A | US - A - 3 073 866 (LESTER N. STANLEY)<br>* Claim 1 *<br>---- | 1 | TECHNICAL FIELDS SEARCHED (Int. Cl. 3)<br>C 07 C 45/00<br>C 07 C 49/00 |

The present search report has been drawn up for all claims

| Place of search | Date of completion of the search | Examiner |
|---|---|---|
| VIENNA | 24-11-1982 | REIF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document